# EUROPEAN PATENT SPECIFICATION

(11) **EP 4 732 195 B1**
(45) Date of publication and mention of the grant of the patent: **09.12.2026**
(21) Application number: 24733607.6
(22) Date of filing: 17.06.2024
(51) Int. Cl.: G06N 3/084, G06T 7/11, G06V 10/82, G16H 30/40, G16H 50/20

(54) **MODIFYING A MACHINE-LEARNING ALGORITHM USING MEASURES OF TIME**
MODIFIZIERUNG EINES MASCHINENLERNALGORITHMUS UNTER VERWENDUNG VON ZEITMESSUNGEN
MODIFICATION D'UN ALGORITHME D'APPRENTISSAGE AUTOMATIQUE À L'AIDE DE MESURES DE TEMPS

(30) Priority: 26.06.2023 RU 2023116660
(43) Date of publication of application: 29.04.2026
(73) Proprietor: Koninklijke Philips N.V., 5656 AE Eindhoven (NL)
(72) Inventor: WEESE, Rolf Jürgen, 5656 AG Eindhoven (NL); FLÄSCHNER, Nick, 5656 AG Eindhoven (NL); WENZEL, Fabian, 5656 AG Eindhoven (NL); EWALD, Arne, 5656 AG Eindhoven (NL); MARKOV, Nikita, 5656 AG Eindhoven (NL); PADALKO, Mikhail, 5656 AG Eindhoven (NL); KOPELOWITZ, Evi, 5656 AG Eindhoven (NL)
(74) Representative: Philips Intellectual Property & Standards
(86) International application number: PCT/EP2024/066764
(87) International publication number: WO 2025/002875

(56) References cited:
- KONTOGIANNI THEODORA ET AL: "Continuous Adaptation for Interactive Object Segmentation by Learning from Corrections", 1 January 2020, SPRINGER, PAGE(S) 579 - 596, XP047565585
- YOO DONGGEUN ET AL: "Learning Loss for Active Learning", 2019 IEEE/CVF CONFERENCE ON COMPUTER VISION AND PATTERN RECOGNITION (CVPR), IEEE, 15 June 2019 (2019-06-15), pages 93 - 102, XP033687105, DOI: 10.1109/CVPR.2019.00018

## Description

### FIELD OF THE INVENTION

The present invention relates to the field of image processing, and in particular to the processing of image data using a machine-learning algorithm.

### BACKGROUND OF THE INVENTION

There is an increasing use of machine-learning algorithms, i.e., algorithms that make use of artificial intelligence concepts, to perform image analysis tasks. Examples of suitable machine-learning algorithms for processing image data are well known in the art, such as neural networks (NNs). One intent of such a machine-learning algorithm is to accurately perform tasks that would otherwise be time-consuming for an adequately trained and/or experienced user/individual to perform.

One particularly advantageous use-case scenario for machine-learning algorithms in processing image data is in clinical environments. In particular, accurate processing of medical image data using a machine-learning algorithm can significantly reduce the time and attention burden on a clinician or caregiver to review the image data. Exemplary image analysis tasks in this field include the detection of a finding for AI-based reporting or the segmentation of a structure, such as the heart chambers, for measurements.

Kontogianni Theodora et Al. "continuous adaptation for interactive object segmentation by learning from corrections", springer, page(s) 579-596 disclose an example of adapting the segmentation model based on user-provided corrections. These corrections are treated as sparse training data, enabling real-time refinement of the model.

Yoo Donggeun et Al. "Learning loss for active learning", 2019 IEEE/CVF Conference on computer vision and pattern recognition (CVPR), IEEE, 15 June 2019, pages 93-102 discloses an example of a system which is trained to estimate the loss of unlabeled data points, enabling the system to identify and prioritize samples that are likely to be misclassified.

There is therefore an ongoing desire to improve the performance (i.e., accuracy) and/or other characteristics of machine-learning algorithms designed to perform an image analysis task.

### SUMMARY OF THE INVENTION

According to examples in accordance with an aspect of the invention, there is provided a computer-implemented method for modifying a machine-learning algorithm configured to process image data to perform an image analysis task.

The computer-implemented method comprises: obtaining at least one measure of time for each of one or more instances of image data, each measure of time being a measure of the time required for an individual to: perform the image analysis task for the instance of image data; or correct, approve or disapprove of the output data produced by the machine-learning algorithm performing the image analysis task on the instance of image data. The computer-implemented method also comprises modifying one or more parameters of the machine-learning algorithm using the at least one measure of time.

The present disclosure proposes an approach for improving the performance of a machine-learning algorithm (that process image data to produce output data) using an amount of time that an individual spends performing a certain interaction with the image data or output data. One of the aims of the proposed approach is to reduce the amount of time that the individual needs to spend with the image and/or output data in order for the image analysis task to be in a form that is approvable by the individual. In some instances, the user provides feedback on the performance of the machine-learning algorithm. Another aim is to predict the required time that a task would take in e.g., segmentation context, and trying to prioritize the tasks.

In the context of the present disclosure, modifying a parameter comprises modifying a value of the parameter, e.g., a value of a learnable parameter, such as a weight of a machine-learning algorithm, a value of a threshold for use with a machine-learning algorithm or the like.

The step of modifying comprises training or retraining the machine-learning algorithm using the one or more measures of time. This process uses the one or more measures of time to weight other measures of loss in (re-) training the machine-learning algorithm.

The step of training or retraining the machine-learning algorithm may comprise: for each instance of image data: obtaining the output data produced by processing the instance of image data using the machine-learning algorithm; obtaining ground-truth output data representing a ground-truth version of the output data representative of the predicted outcome of the image analysis task; determining a measure of loss between the output data and the ground-truth output data; and weighting the measure of loss using the at least one measure of time for the instance of image data; and training or retraining the machine-learning algorithm using all the weighted measures of loss.

In this way, the machine-learning algorithm is trained using the measure(s) of time as a mechanism to weight measures of loss between ground-truth output data and predicted output data. This configures the training process of the machine-learning algorithm to focus or target improving the algorithms performance in performing the image analysis task on data that requires a significant amount of time for an individual to review and/or perform themselves. This approach thereby, at least in some instances, provides a machine-learning algorithm with significantly improved characteristics in processing data that targets complex and/or time-consuming tasks for an individual.

In some examples, the method further comprises normalizing the obtained at least one measure of time before weighting, for each instance of image data, the measure of loss.

In some examples, for each instance of image data, at least one measure of time for the instance of image data is a measure of the time required for the individual to perform the image analysis task for the instance of image data; and the ground-truth output data is data produced by the individual performing the image analysis task for the instance of image data.

This approach allows the necessary ground-truth generation procedure to be used in producing the measures of time, avoiding or reducing the amount of separate dedicated time required to generate the data required to carry out the proposed approach.

The step of determining a measure of loss may comprise determining a measure of cross-entropy loss and/or a measure of dice-loss between the output data and the ground-truth output data.

In some examples, at least one measure of time for each of one or more instances of image data comprises a measure of the time taken for the individual to supplement output data produced by the machine-learning algorithm after processing the instance of image data to perform the image analysis task on the instance of image data.

In some examples, at least one measure of time for each of one or more instances of image data comprises a measure of the time taken for the individual to approve output data produced by the machine-learning algorithm after processing the instance of image data to perform the image analysis task on the instance of image data.

In some examples, at least one measure of time for each of one or more instances of image data comprises a measure of the time taken for the individual to remove at least a portion of output data produced by the machine-learning algorithm after processing the instance of image data to perform the image analysis task on the instance of image data.

In some examples, the image analysis task is an image classification task; the machine-learning algorithm uses a threshold to predict whether or not the image data belongs to a classification; and modifying one or more parameters using the one or more measures of time to modify the value of the threshold. In some instances, the classification task can be also performed by a dedicated classification module.

In some examples, the one or more instances of image data comprises at least 50 instances of image data.

In some examples, the machine-learning algorithm is a neural network. A neural network is particularly suited to performing an image analysis task, and provides improved accuracy and/or robustness over other forms of machine-learning algorithms.

The image data may be medical image data. The analysis of medical image data is a particularly useful use-case scenario for using machine-learning algorithms, as medical image data is complex and has historically required significant user input and/or expertise to accurately assess.

The image analysis task is a(n image) segmentation task.

There is also proposed a computer program product comprising computer program code means which, when executed on a computing device having a processing system, cause the processing system to perform all of the steps of any herein disclosed method.

There is also proposed a processing system for modifying a machine-learning algorithm configured to process image data to perform an image analysis task.

The processing system is configured to: obtain at least one measure of time for each of one or more instances of image data, each measure of time being a measure of the time required for an individual to perform the image analysis task for the instance of image data; or correct, approve or disapprove of the output data produced by the machine-learning algorithm performing the image analysis task on the instance of image data; and modify one or more parameters of the machine-learning algorithm using the one or more measures of time.

Any herein disclosed processing system may be appropriately adapted to carry out the functions of any herein disclosed method, and vice versa. The skilled person would be similarly capable of adapting a computer program product to carry out any herein disclosed method.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a flowchart illustrating a proposed approach;
Fig. 2 is a flowchart illustrating a step for use in a proposed approach; and
Fig. 3 is a flowchart illustrating an alternative step for use in a proposed approach.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for integrating a user interaction time into the training and/or adaptation of one or more parameters for a machine-learning algorithm designed to perform a particular image analysis task.

The present disclosure proposes an approach in which a measured user interaction time is integrated into the training or modification of machine-learning algorithm designed to accomplish a specific image analysis task. In particular, a measured user interaction time can be used as an additional criterion during training of the machine-learning algorithm and/or for defining the operating point (e.g., one or more thresholds) of the machine-learning algorithm. This can be used for any medical or non-medical application. For instances, in some embodiments the main use case may be in segmentation, for instances in contouring of each slices or during interpolation of contouring tasks, e.g., slices having no contouring are interpolated to the slices that have contouring. Typically, neural networks and other types of machine learning approaches are trained to optimally predict the desired result with high accuracy, assuming that user interaction time is reduced more the more accurate the neural network result is. On the other hand, within one class of tasks, some results are more time consuming for the user to generate or correct than other results.

For instance, due to the slice thickness, segmentation of the left or right ventricle in cine MR images is usually done by contouring the desired structure slice by slice. When doing this for ground truth generation, it is proposed in some instances to measure the time that is needed to create the contour per slice (e.g., time from defining first contour point to closing a contour for all contours on the slice). When no contour is generated (i.e., a structure not visible in the slice), a minimum user interaction time is recorded (that corresponds e.g. to the action "remove contour on the slice"). In some further examples, the goal is to account for the time to initially analyze a case at hand, normalize the time per finding to correspond on average with user interaction time for subsequently entered findings, and improve the segmentation and/or contouring and/or workflow tasks. This and other clinical and non-clinical applications will be apparent from the following description of the invention.

In one aspect, the inventon provides for a method and/or a system for processing image data to perform an image analysis task and produce output data representative of a predicted outcome of the image analysis task, wherein the method is comprising obtaining at least one measure of time for one or more instance of image data, the measure of time being a measure of the time required for an individual to: perform the image analysis task for the instance of image data; and/or correct, approve and/or disapprove of the output data produced by the machine-learning algorithm performing the image analysis task on the instance of image data; and modifying one or more parameters of the machine-learning algorithm using the at least one measure of time.

As an example, training samples that require more user interaction time might get a higher weight in the training objective or loss function than training samples that require little/less user interaction time. As another/further example, a training objective / loss function predicting the time savings in dependence of the measured user interaction time might be added to any existing training objectives. As a yet other/further example, the operating point or threshold(s) for, e.g., a classifier might be determined based on predicted time savings. This way, user interaction time for an AI supported image analysis workflow is improved. For instance, training samples that require more user interaction time might get a higher weight in the training objective / loss function than training samples that require little user interaction time. In addition or alternatively, a training objective / loss function predicting the time savings in dependence of the measured user interaction time might be added to the typically used training objectives / loss functions. Furthermore, the optimal operating point for, e.g., a classifier might be determined based on predicted time savings. In that way, user interaction time for an AI supported image analysis workflow is optimized.

In at least some embodiments, the invention proposes to measure user interaction time to accomplish a specific image analysis task (detection of a finding for machine learning-based reporting; segmentation of a structure for measurements) and use the measured time as an additional criterium during machine learning model (e.g., neural network) training or for defining the optimal operating point. For instance, training samples that require more user interaction time might get a higher weight in the training objective / loss function than training samples that require little user interaction time. In addition or alternatively, a training objective / loss function predicting the time savings in dependence of the measured user interaction time might be added to the typically used training objectives. Furthermore, the optimal operating point for, e.g., a classifier might be determined based on predicted time savings. One of the benefits may be that the user interaction time for an image analysis workflow is optimized.

Example image analysis tasks includes: identification or detection of one or more findings in image data; segmentation of image data; classification of image data (which may include classification of a finding or pixel-wise classification and/or or region-wise classification for performing a segmentation function); measuring one or more dimensions of an element represented in image data; and so on. Another example of an image analysis task is the manipulation of one or more control points for a contour representing a segmentation result.

One example of an image analysis task is a segmentation task performed using a classification procedure. In particular, the image data may be sub-divided into a plurality of regions (e.g., pixels or groups of pixels). Each region, e.g., each pixel, may be classified using a classification procedure. This effectively performs a segmentation task using regional or region-based classification.

The present disclosure thereby provides a technique for training or retraining a machine-learning algorithm and/or updating an operating point for the machine-learning algorithm.

A machine-learning algorithm is any self-training algorithm that processes input data in order to produce or predict output data. Here, the input data comprises image data (e.g., an image) and the output data comprises an outcome or result of an image analysis task (e.g., a segmentation result, a classification result, a measuring task, and so on).

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

In some examples, e.g., if the machine-learning algorithm performs a classification task, the output of the final layer may be compared to one or more thresholds to determine the classification. The value of the threshold may be defined in the training process or separately, e.g., depending upon a desired level of accuracy (e.g., a desired true positive rate, a desired false positive rate and so on).

Methods of training a machine-learning algorithm are well known, as briefly explained by, amongst others, Wang, H., et al. "Machine learning basics." Deep learning (2016): 98-164 or Bhavsar, Hetal, and Amit Ganatra. "A comparative study of training algorithms for supervised machine learning." International Journal of Soft Computing and Engineering (IJSCE) 2.4 (2012): 2231-2307.

Typically, training methods comprise obtaining a training dataset, comprising instances of input data and corresponding instances of ground truth output data (also known as instances of training output data). An initialized machine-learning algorithm is applied to each instance of input data to generate an instance of predicted output data. Thus, each instance of predicted output data corresponds to an instance of ground truth output data. For each instance of predicted output data, a measure of error or loss is determined between the instance of predicted output data and the instance of ground truth output data. The determined errors are used to modify the machine-learning algorithm. In particular, the machine-learning algorithm may be iteratively modified (and the measure of error iteratively updated) until the (total) error converges, and the instances of predicted output data are sufficiently similar (e.g. ±1%) to the instances of predicted input data. This is commonly known as a supervised learning technique. Preferably, the interactive tool for ground truth generation should be the same tool that is used in combination with the results (e.g., contours may be initialized using the previously trained AI algorithm).

For example, where the machine-learning algorithm is formed from a neural network, (weightings of) the mathematical operation of each neuron may be modified until the (total) error converges. Known methods of modifying a neural network include gradient descent, backpropagation algorithms and so on.

Known measures of error include the measures of loss produced by performing an L1 or L2 loss function, or any measure of loss produced using any loss function mentioned by Jadon, Shruti. "A survey of loss functions for semantic segmentation." 2020 IEEE conference on computational intelligence in bioinformatics and computational biology (CIBCB). IEEE, 2020; Wang, Qi, et al. "A comprehensive survey of loss functions in machine learning." Annals of Data Science (2020): 1-26.; and/or Ciampiconi, Lorenzo, et al. "A survey and taxonomy of loss functions in machine learning." arXiv preprint arXiv:2301.05579 (2023).

The instances of input data correspond to example instances of image data. The instances of output data correspond to example results of an image analysis task.

The present disclosure provides mechanisms for setting or defining the value(s) for one or more parameters of a machine-learning algorithm, e.g., controlling the value of any weight(s) in the machine-learning algorithm and/or any thresholds. Specific examples will be provided later in this disclosure.

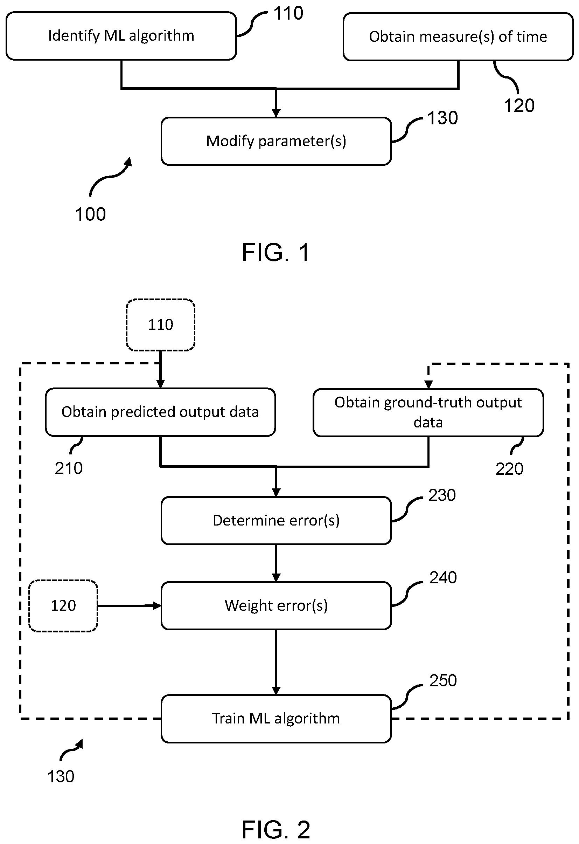

Fig. 1 is a flowchart illustrating a computer-implemented method 100 for modifying a machine-learning algorithm configured to process image data to perform an image analysis task. The machine-learning algorithm will produce output data representative of a predicted outcome of the image analysis task (on the processed image data).

The computer-implemented method may comprise a step 110 of identifying the machine-learning algorithm configured to process image data to perform the image analysis task and produce output data representative of a predicted outcome of the image analysis task.

Thus, step 110 comprises identifying the machine-learning algorithm that is to perform the image analysis task on image data. The machine-learning algorithm may be a trained machine-learning algorithm (i.e., already trained to perform the task) or an untrained machine-learning algorithm (i.e., an initialized machine-learning algorithm that is to be trained to perform the task).

Step 110 is not essential to the method. Rather, the method may be used to process an already identified, defined or otherwise relevant machine-learning algorithm.

The method 100 also comprises a step 120 of obtaining at least one measure of time for each of one or more instances of image data. Each measure of time is a measure of the time required for an individual to either perform the image analysis task for the instance of image data; or (after being provided with the output data produced by the machine-learning algorithm performing the image analysis task on the instance of image data) correct, approve or disapprove the output data. The measure may, for instance, be defined in seconds, clock cycles or any other suitable measure of time.

Of course, it will be appreciated that if a measure of time is a measure of the time required for the individual to correct, approve or disapprove output data, then the step 120 may further comprise processing, for each instance of image data, processing the instance of image data using the machine-learning algorithm to produce the output data.

The one or more instances of image data may, for instance, comprise at least twenty instances, e.g., at least fifty instances, e.g., at least one hundred instances.

The method 100 also comprises a step 130 of modifying one or more parameters of the machine-learning algorithm using the one or more measures of time. Put another way, step 130 comprises modifying, for each of one or more parameters of the machine-learning algorithm, a value of the parameter.

Step 130 may, for instance, comprise using the at least one measure of time to weight a particular measure of loss, for training or retraining the image analysis task. This approach allows for a time required for a user to perform/correct the task to be taken into account when modifying the machine-learning algorithm.

As another example, the at least one measure of time may define a threshold for a classification task.

A number of working examples that employ the principle(s) outlined above are hereafter described.

A first set of working examples make use of measures of time for (e.g., initial) training the machine-learning algorithm. In particular, the measures of time are used to define and/or weight one or more measures of loss used in training the machine-learning algorithm.

In a first one of the first set of working examples, each measure of time is a measure of how long it takes the skilled person to perform the image analysis task (on a different image). More particular, each measure of time is a measure of how long it takes the skilled person to perform the image analysis on a different instance of image data without input from a machine-learning algorithm. Thus, each measure of time may be the length of time between the individual being presented with the instance of image data and the time at which the individual inputs or submits the corresponding outcome of the image analysis task, e.g., provides an instance of ground-truth data.

In one example scenario, the image analysis task is to perform segmentation of a medical image, such as a segmentation of the left or right ventricle in a MR image of the heart. In this scenario, each measure of time may be a measure of how long it takes an individual to identify or create a contour of the desired structure within a particular image. If no contour is generated, then the measure of time may be set to a minimum time.

In another example scenario, the image analysis task is to perform classification of a medical image, such as classification of whether or not a particular pathology or feature is visible in the image, a classification of the view or contents of the image and so on. The classification may be on a whole-image basis, on a region of the image basis and/or on a pixel-by-pixel basis (an example of a region of the image basis). In this scenario, each measure of time may be a measure of how long it takes an individual to perform and/or input the classification(s) of the image.

Other suitable scenarios and image analysis tasks will be readily apparent to the appropriately skilled person.

In a second one of the first set of working examples, each measure of time is a measure of how long it takes the skilled person to correct, approve or disapprove output data produced by the machine-learning algorithm performing the image analysis task on the instance of image data. Thus, in this approach, the measure of time may measure how long it takes the skilled person or individual to assess and/or (if necessary) change the output data produced by the machine-learning algorithm.

This approach helps identify weak areas of the machine-learning algorithm, as weak areas will take longer for the skilled person to approve, disapprove and/or correct compared to stronger areas.

In one example scenario of this second one in the first set of working examples, the image analysis task is to perform segmentation on an instance of image data. For example, the segmentation may produce a contour with control points of a desired anatomical element (e.g., the left or right ventricle in a MR image slice). The contour produced by the machine-learning algorithm may be manually correctable (by an individual) by selecting a control point and moving it to the desired position. It is proposed to measure the time taken for the skilled to perform a correction of the contour, produced by the machine-learning algorithm, as the measure of time.

In some examples, the machine-learning algorithm is configured to perform manipulation of control points of a contour that defines a segmentation result of the instance of image data. In this approach, the image analysis task may be a task of manipulating a control point until it is positioned at the perimeter or boundary of a region to be segmented. Put another way, the machine-learning algorithm may be designed or intended to predict the movement of a control point to a new position. The measure of time may be a measure of time for an individual to correct a proposed movement of the control point. In this scenario, the measure of time may be a measure between the end of a previous manual movement of a control point and the end of the manual movement of a current control point.

In another example scenario of this second one of the first set of working examples, the machine-learning algorithm is configured to determine the presence or absence of one or more findings (e.g., pathologies or anatomical structures/element) in the processed instance of image data. The measure of time may, for instance, be a measure of time taken for the individual to approve, correct or disapprove of the determined one or more findings.

The outcome of the performance of the image analysis task by the individual may define ground-truth data used for training the machine-learning algorithm. Thus, each measure of time may effectively be a measure of how long it takes the individual to generate an instance of ground-truth data from an instance of image data. The instance of image data and the instance of ground-truth data may effectively form a training input data entry and training output data entry pair, which the machine-learning algorithm uses to undergo training.

In any above-described examples, to account for the time for an individual to initially look at the instance of image data and/or the output of the machine-learning algorithm (where relevant), the mean and standard deviation of the measures of time may be normalized to correspond on average with historic measures of time.

Fig. 2 illustrates one approach to performing step 130, which can be used in any of the first set of working examples.

The illustrated embodiment of step 130 comprises a step 210 of, for each instance of image data, obtaining the output data produced by processing the instance of image data using the machine-learning algorithm - which defines an instance of predicted output data.

Step 210 may, for instance, be performed by obtaining the one or more instances of image data (e.g., from a memory or database) and processing each instance of image data using the machine-learning algorithm. In another example, step 210 is performed by retrieving or receiving the output data (for each instance of image data) from an external system or memory.

The illustrated embodiment of step 130 also comprises a step 220 of, for each instance of image data, obtaining ground-truth output data representing a ground-truth version of the output data representative of the predicted outcome of the image analysis task.

As previously mentioned, the ground-truth output data may be (previously) generated by an appropriately trained or experienced individual performing the image analysis task on the instance of image data and indicating or inputting the outcome of the image analysis task, which is then stored as the ground-truth output data.

The illustrated embodiment of step 130 also comprises a step 230 of, for each instance of image data, determining a measure of loss between the output data and the ground-truth output data. A wide variety of potential measures of loss will be apparent to the skilled person, and may depend upon the precise nature of the image analysis task and/or the data format of the (ground-truth and/or predicted) output data.

As an example, where the output data takes the form of an image (e.g., a segmented image), then suitable examples for a measure of loss include a measure of cross-entropy loss and/or a measure of dice-loss between the output data and the ground-truth output data. Other examples are known to the skilled person, such as measures of loss produced by performing an L1 or L2 loss function, or any measure of loss produced using any loss function mentioned by Jadon, Shruti. "A survey of loss functions for semantic segmentation." 2020 IEEE conference on computational intelligence in bioinformatics and computational biology (CIBCB). IEEE, 2020; Wang, Qi, et al. "A comprehensive survey of loss functions in machine learning." Annals of Data Science (2020): 1-26.; and/or Ciampiconi, Lorenzo, et al. "A survey and taxonomy of loss functions in machine learning." arXiv preprint arXiv:2301.05579 (2023)"".

The illustrated embodiment of step 130 also comprises a step 240 of, for each instance of image data, weighting the measure of loss using the at least one measure of time for the instance of image data.

As an example, each measure of time may be associated with a single instance of image data (representing a measure of the time taken for an individual to perform or input the outcome of the image analysis task). All obtained measures of time may be normalized, e.g., to a value between 0 and 1. Each measure of loss may then be multiplied by its corresponding measure of time to appropriately weight the measures of loss.

In some examples, e.g., before normalization, outlying measures of time may be clipped, e.g., the top X% may be clipped or set to a same value, where X is any suitable percentage. For example, X may be less than 10, e.g., less than 5. Preferably, X is greater than 1, e.g., greater than 2. For example, X may be equal to 3.

In this way, each measure of loss is weighted in step 240 based on its corresponding measure of time.

Step 130 then comprises a step 250 of training or retraining the machine-learning algorithm using all the weighted measures of loss. Step 250 may, for instance, comprise iteratively modifying the value(s) of the parameter(s) of the machine-learning algorithm using known approaches. It may be necessary to iteratively repeat steps 210-240 throughout the training performed by step 250, e.g., to update the weighted measures of loss. In particular, at least steps 210, 230 and 240 may need to be repeated for each modification to the machine-learning algorithm performed in step 250. This is illustrated using dashed lines.

Suitable approaches for training or retraining a machine-learning algorithm using one or more measures of loss are well known. The proposed weighted measure(s) of loss can be readily integrated into such procedures.

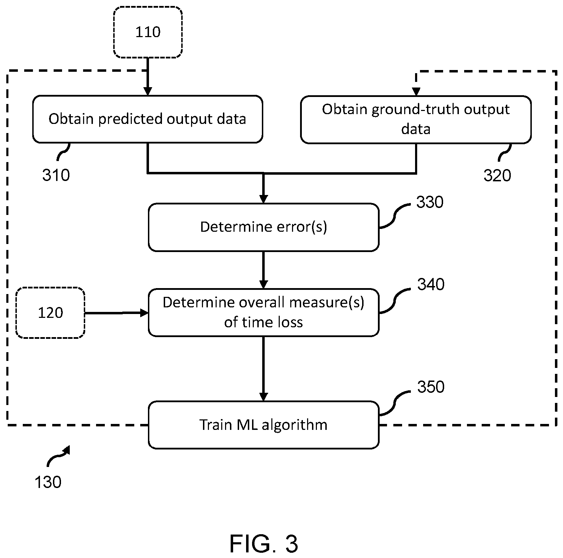

Fig. 3 illustrates an alternative approach to performing step 130, which can be used in any of the first set of working examples.

The illustrated embodiment of step 130 comprises a step 310 of, for each instance of image data, obtaining the output data produced by processing the instance of image data using the machine-learning algorithm - which defines an instance of predicted output data. Approaches for performing such a step have been previously described.

The illustrated embodiment of step 130 also comprises a step 320 of, for each instance of image data, obtaining ground-truth output data representing a ground-truth version of the output data representative of the predicted outcome of the image analysis task.

As previously mentioned, the ground-truth output data may be (previously) generated by an appropriately trained or experienced individual performing the image analysis task on the instance of image data and indicating or inputting the outcome of the image analysis task, which is then stored as the ground-truth output data.

The illustrated embodiment of step 130 also comprises a step 330 of, for each instance of image data, determining a measure of loss between the output data and the ground-truth output data. A wide variety of potential measures of loss will be apparent to the skilled person, and may depend upon the precise nature of the image analysis task and/or the data format of the (ground-truth and/or predicted) output data.

Example approaches for determining a measure of loss are well known in the art, and some examples have been previously described.

The illustrated embodiment of step 130 also comprises a step 340 of determining an overall measure of predicted time loss using the measure(s) of time. The overall measure of predicted time loss may act as (for training of the machine-learning algorithm) as an objective / loss term that predicts the user interaction time and accumulates it over instances of image data.

Optionally, step 340 comprises normalizing the measures of time, e.g., to an interval [0,1]. This improves robustness of the proposed approach.

The overall measure of predicted time loss may be determined by summing those (normalized) measures of time that correspond to a measure of loss that meets one or more predetermined criteria. For instance, only those measures of time corresponding to a measure of loss falling within a predetermined range may contribute to (i.e., be summed to produce) the overall measure of predicted time loss. The predetermined range may, for instance, define when the measure of loss is within acceptable or desirable bounds.

For instance, if the measure of loss is a (normalized) dice score (where a higher dice score is desirable), the corresponding measure of time may form part of the sum for the overall measure of predicted time loss only when the dice score is above a first threshold (e.g., 0.9). If below this threshold, then the measure of time does not form part of the sum.

In some embodiments, based on the threshold the number of true positives of the machine learning, true negatives, false positives and false negatives can vary. Using the average times, the threshold can now be defined to minimize the resulting user interaction time. Additionally or alternatively, an interval between [lower_threshold, upper_threshold] may be defined for which no prediction is shown, i.e., when the prediction is neither close to 1 or to 0, indicating a large machine learning, e.g., neural network, uncertainty. Similarly, when training e.g., a neural network, an additional objective / loss function might be added that evaluates the user interaction time in dependence of the actual network configuration for a specific finding, a threshold, true negative, false positives, false negatives, as predicted by the actual network configuration and threshold, and the average times can vary.

In some embodiments, for weighting the individual terms of the objective / loss function with the measured user interaction time, e.g. 3% largest times are clipped and the resulting times are scaled to the interval [0,1]. Each slice-based term of the cross-entropy loss and the dice loss are weighted with the user interaction times after mapping. This mechanism emphasizes image slices during training that need more time for interactive correction. In addition or alternatively, an objective / loss term is added that predicts the user interaction time and accumulates it over all image slices. In at least some embodiments, the user interaction times are mapped to the interval [0,1]. As a representaive example, for a slice with no reference contour, the minimum user interaction time after mapping is added when the actual network configuration labels a voxel in the slice. In some embodiments, for a slice with reference contour, the mapped user interaction time is added when the dice score is below a threshold (e.g. 0.9) and 0 is added when the dice score is above a threshold (e.g. 0.95). In between the mapped user interaction time is linearly interpolated. By this way, the predicted user interaction time can be minimized.

In some examples:
the (normalized) measure of time does not form part of the sum for the overall measure of predicted time loss if the corresponding measure of loss falls within a first range;
the (normalized) measure of time forms part of the sum for the overall measure of predicted time loss if the corresponding measure of loss falls within a second, different range, where the first and second ranges are separated by a third range; and
the (normalized) measure of time is linearly interpolated between a value of 0 and the (original) value of the (normalized) measure of time if the corresponding measure of loss falls within the third range. A linear interpolation means that the (normalized) measure of time is weighted based on its position within the third range (where closer to the first range means that the measure of time is adjusted to be closer to zero and closer to the second range means that the measure of time is adjusted to closer to the (original) value of the measure of time.

Step 130 then comprises a step 350 of training or retraining the machine-learning algorithm using the overall measure of predicted time loss. In particular, step 250 may comprise (re)training the machine-learning algorithm to reduce or minimize the value of the overall measure of predicted time loss. This mechanism aims at minimizing the (predicted) user interaction time during training. Step 350 may, for instance, comprise iteratively modifying the value(s) of the parameter(s) of the machine-learning algorithm using known approaches.

In a similar way to the previously described approach, it may be necessary to iteratively repeat steps 310-340 throughout the training performed by step 350, e.g., to update the weighted measures of loss. In particular, at least steps 310, 330 and 340 may need to be repeated for each modification to the machine-learning algorithm performed in step 350.

In some variants of the approach described with reference to Fig. 3, step 340 is repeated for a plurality of different measures of time. In this way, multiple overall measures of predicted time loss may be produced. Step 350 may be appropriately adapted to take account of using the multiple overall measures of predicted time loss (e.g., by summing them together and aiming to minimize the summed output).

As an example, it has previously been explained how a measure of loss may be a measure of how long it takes an individual to: perform the image analysis task for the instance of image data; or, after being provided with the output data produced by the machine-learning algorithm performing the image analysis task on the instance of image data, correct, approve or disapprove the output data.

In some examples, measures of time for performing the image analysis task, or correcting, approving and disapproving the output data, may be considered separately. Thus, step 340 may comprise determining a first overall measure, a second overall measure, a third overall measure and/or a fourth overall measure.

The first overall measure is an overall measure of predicted time for the individual to perform the image analysis task. This can be calculated by summing any measures of time for the individual to perform the image analysis task.

The second overall measure is an overall measure of predicted time for correcting the output data. This can be calculated by summing any measures of time for an individual to correct or change an outcome of an image analysis task performed by the machine-learning algorithm.

The third overall measure is an overall measure of predicted time loss for approving the output data. This can be calculated by summing any measures of time for an individual to approve of an outcome of an image analysis task performed by the machine-learning algorithm.

The fourth overall measure is an overall measure of predicted time loss for disapproving the output data. This can be calculated by summing any measures of time for an individual to disapprove of an outcome of an image analysis task performed by the machine-learning algorithm.

Each overall measure may be produced by only adding those measures of time that meet the (additional) condition that they represent a measure of time corresponding to relevant act (of performing the image analysis task or of correcting, approving or disapproving).

Step 350 may be adapted accordingly. In particular, step 350 may comprise training or retraining using the first, second, third and/or fourth measures outlined above, e.g., to reduce or minimize the values of the first, second, third or fourth measures.

Of course, the two approaches described with reference to Figs. 2 and 3 may be combined. In this way, each measure of loss for each instance of image data may be weighted and an overall measure of predicted time loss may be generated and used in the training or retraining of the machine-learning algorithm.

In the approaches described with reference to Figs. 2 and 3, the parameter(s) of the machine-learning algorithm that is modified by step 130 is weights or other parameters within the machine-learning algorithm itself.

In another approach, the parameter(s) of the machine-learning algorithm includes an output threshold.

In particular, the image analysis task may be a classification task in which the machine-learning algorithm uses a threshold to predict whether or not the image data (or a region thereof, such as a pixel or superpixel) belongs to a classification. This can achieve classification of the entire image data, classification of each of a plurality of regions of the image data and/or segmentation (e.g., as pixels belonging to a same classification can represent a segmentation result).

In this approach, modifying, for each of one or more parameters of the machine-learning algorithm, a value of the parameter may comprise using the one or more measures of time to modify the value of the threshold.

It will be appreciated that, based on the value of the threshold, the number of true positives NTP, true negatives NTN, false positives NFP and false negatives NFN varies. Using the determined measure(s) of time, the threshold may now or further be defined to achieve a desired time for an individual.

In any above-described example, the measures of time may represent the time required for a single individual to perform the relevant act/task (of performing the image analysis task or of correcting, approving or disapproving). This provides a user-specific and user bespoke approach to modifying the machine-learning algorithm, i.e., resulting in a user-specific or bespoke machine-learning algorithm.

In other examples, the measures of time may represent the time required for any one of a plurality of individuals to perform the relevant act/task (of performing the image analysis task or of correcting, approving or disapproving). This provides a machine-learning algorithm that is more adapted for a general population of people. The plurality of individuals may, for instance, be a restricted population (e.g., individuals associated with a particular clinical setting, such as staff at a particular hospital) or an unrestricted population (e.g., any individual permitted to produce training data for the machine-learning algorithm).

The machine-learning algorithm produced using any above-described approach can be repurposed for use in a separate system (to the system that modifies the machine-learning algorithm). This separate system may, for instance, employ its own approach for modifying the machine-learning algorithm, e.g., employ another instance or embodiment of the proposed approach.

The skilled person would be readily capable of developing a processing system for carrying out any herein described method. Thus, each step of the flow chart may represent a different action performed by a processing system, and may be performed by a respective module of the processing system.

Embodiments may therefore make use of a processing system. The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method.

There is also proposed a non-transitory storage medium that stores or carries a computer program or computer code that, when executed by a processing system, causes the processing system to carry out any herein described method.

In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

In the claims, the word "comprising" does not exclude other elements or steps, and the indefinite article "a" or "an" does not exclude a plurality. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

A single processor or other unit may fulfill the functions of several items recited in the claims. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems.

In a networked deployment, the computer system operates in the capacity of a server, or as a client user computer in a server-client user network environment, or as a peer computer system in a peer-to-peer or distributed network environment. The computer system can also be implemented as or incorporated into various devices, such as a server or another type of computer such as a workstation that includes a controller, a stationary computer, a mobile computer, a personal computer (PC), a laptop computer, a tablet computer, or any other machine capable of executing a set of software instructions sequentially or non-sequentially that specify actions to be taken by that machine. The computer system can be incorporated as an integrated system part of a larger system that includes additional devices. In an embodiment, the computer system can be implemented using electronic devices that provide voice, video, or data communication possibilities. Further, while the computer system is illustrated in the singular, the term "system" shall also be taken to include any collection of systems or sub-systems that individually or jointly execute a set or multiple sets, of software instructions to perform one or more computer functions.

The computer system may also include a processor. The processor executes instructions to implement some, or all aspects of methods and processes described herein. The processor is tangible and non-transitory. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The processor is an article of manufacture and/or a machine component. The processor is configured to execute software instructions to perform functions as described in the various embodiments herein. The processor may be a general-purpose processor or may be part of an application specific integrated circuit (ASIC). The processor may also be a microprocessor, a microcomputer, a processor chip, a controller, a microcontroller, a digital signal processor (DSP), a state machine, or a programmable logic device, a logical circuit, including a programmable gate array (PGA), such as a field programmable gate array (FPGA), or another type of circuit that includes discrete gate and/or transistor logic. The processor may be a central processing unit (CPU), a graphics processing unit (GPU), or both. Additionally, any processor described herein may include multiple processors, parallel processors, or both. Multiple processors may be included in, or coupled to, a single device or multiple devices. The processor can include one or more internal levels of cache, and a bus controller or bus interface unit to direct interaction with a bus. The term "processor" as used herein encompasses an electronic component able to execute a program or machine executable instruction. References to a computing device comprising "a processor" should be interpreted to include more than one processor or processing core, as in a multi-core processor. A processor may also refer to a collection of processors within a single computer system or distributed among multiple computer systems. The term computing device should also be interpreted to include a collection, or network, of computing devices each including a processor or processors. Programs have software instructions performed by one or multiple processors that may be within the same computing device or which may be distributed across multiple computing devices. Further, the software instructions, when executed by the processor, perform one or more steps of the methods and processes as described herein.

The computer system further includes a main memory and a static memory, where memories in the computer system communicate with each other and the processor via a bus. Either or both main memory and the static memory may be considered representative examples of the memory of the controller, and store instructions used to implement some, or all aspects of methods and processes described herein. Memories described herein are tangible storage mediums for storing data and executable software instructions and are non-transitory during the time software instructions are stored therein. The main memory and the static memory are articles of manufacture and/or machine components. The main memory and the static memory are computer-readable mediums from which data and executable software instructions can be read by a computer (or e.g., the processor). Each of the main memory and the static memory may be implemented as one or more of random access memory (RAM), read only memory (ROM), flash memory, electrically programmable read only memory (EPROM), electrically erasable programmable read-only memory (EEPROM), registers, a hard disk, a removable disk, tape, compact disk read only memory (CD-ROM (Compact Disk - Read Only Memory)), digital versatile disk (DVD), floppy disk, Blu-ray disk, or any other form of storage medium known in the art. The memories may be volatile or non-volatile, secure and/or encrypted, unsecure and/or unencrypted.

The illustrations of the embodiments described herein are intended to provide a general understanding of the structure of the various embodiments. The illustrations are not intended to fully describe all the elements and features of the disclosure described herein. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the scope of the present disclosure. Thus, to the maximum extent allowed by law, the scope of the present disclosure is to be determined by the broadest permissible interpretation of the following claims and their equivalents and shall not be restricted or limited by the foregoing detailed description. Any reference signs in the claims should not be construed as limiting the scope.

## Claims

1. A computer-implemented method (100) to process image data to perform an image analysis task and produce output data representative of a predicted outcome of the image analysis task, the method comprising:
obtaining at least one measure of time (120) for one or more instance of image data, the measure of time being a measure of the time required for an individual to:
perform the image analysis task for the instance of image data; and/or
correct, approve and/or disapprove of the output data produced by the machine-learning algorithm performing the image analysis task on the instance of image data; and
modifying one or more parameters of the machine-learning algorithm using the at least one measure of time (130), wherein the step of modifying comprises training or retraining the machine-learning algorithm using the one or more measures of time, wherein the image data is an image, the image analysis task is a segmentation task, and the output data comprises a segmentation of the image and the step of training or retraining the machine-learning algorithm comprises:
obtaining the output data produced by processing the instance of image data using the machine-learning algorithm (210);
obtaining ground-truth output data representing a ground-truth version of the output data representative of the predicted outcome of the image analysis task (220);
determining a measure of loss between the output data and the ground-truth output data (230);
weighting the measure of loss using the at least one measure of time for the instance of image data (240); and
training or retraining the machine-learning algorithm using all the weighted measures of loss (250).

2. The method of claim 1 further comprising normalizing the obtained at least one measure of time before weighting, for instance of image data, the measure of loss (340).

3. The method of claim 2, wherein, for instance of image data:
at least one measure of time for the instance of image data is a measure of the time required for the individual to perform the image analysis task for the instance of image data; and
the ground-truth output data is data produced by the individual performing the image analysis task for the instance of image data.

4. The method of claim 2 or 3, wherein the step of determining a measure of loss comprises determining a measure of cross-entropy loss and/or a measure of dice-loss between the output data and the ground-truth output data (230).

5. The method of any one of claims 1 to 4, wherein the at least one measure of time for each of one or more instances of image data comprises a measure of the time taken for the individual to supplement output data produced by the machine-learning algorithm after processing the instance of image data to perform the image analysis task on the instance of image data.

6. The method of any one of claims 1 to 5, wherein the at least one measure of time for instance of image data comprises a measure of the time taken for the individual to approve output data produced by the machine-learning algorithm after processing the instance of image data to perform the image analysis task on the instance of image data.

7. The method of any one of claims 1 to 6, wherein the at least one measure of time for instance of image data comprises a measure of the time taken for the individual to remove at least a portion of output data produced by the machine-learning algorithm after processing the instance of image data to perform the image analysis task on the instance of image data.

8. The method of any one of claims 1 to 7, wherein:
the image analysis task is an image classification task;
the machine-learning algorithm uses a threshold to predict whether or not the image data belongs to a classification; and wherein
modifying one or more parameters of the machine-learning algorithm comprises using the one or more measures of time to modify the value of the threshold (130).

9. The method of any one of claims 1 to 8, wherein the instance of image data comprises at least 50 instances of image data.

10. The computer-implemented method of any one of claims 1 to 9, wherein the machine-learning algorithm is a convolutional neural network.

11. A computer program comprising computer program code means which, when executed on a computing device having a processing system, cause the processing system to perform steps of the method according to any one of claims 1 to 10.

12. A processing system for modifying a machine-learning algorithm configured to process image data to perform an image analysis task and produce output data representative of a predicted outcome of the image analysis task,
the processing system being configured to:
obtain at least one measure of time for one or more instance of image data, each measure of time being a measure of the time required for an individual to:
perform the image analysis task for the instance of image data; and/or
correct, approve or disapprove of the output data produced by the machine-learning algorithm performing the image analysis task on the instance of image data; and
modify one or more parameters of the machine-learning algorithm using the one or more measures of time, wherein the modify comprises to train or retrain the machine-learning algorithm using the one or more measures of time,
wherein the image data is an image, the image analysis task is a segmentation task, and the output data comprises a segmentation of the image, and wherein the train or retrain the machine-learning algorithm is configered to:
obtain the output data produced by processing the instance of image data using the machine-learning algorithm;
obtain ground-truth output data representing a ground-truth version of the output data representative of the predicted outcome of the image analysis task;
determine a measure of loss between the output data and the ground-truth output data; and
weigh the measure of loss using the at least one measure of time for the instance of image data (240); and
train or retrain the machine-learning algorithm using all the weighted measures of loss.

## Patentansprüche

1. Computerimplementiertes Verfahren (100) zum Verarbeiten von Bilddaten, um eine Bildanalyseaufgabe durchzuführen und Ausgabedaten, die repräsentativ für ein vorhergesagtes Ergebnis der Bildanalyseaufgabe sind, zu erzeugen, wobei das Verfahren Folgendes umfasst:
Erhalten mindestens eines Zeitdauermaßes (120) für eine oder mehrere Instanzen von Bilddaten, wobei das Zeitdauermaß ein Maß für die Zeit ist, die eine Person für Folgendes benötigt:
Durchführen der Bildanalyseaufgabe für die Instanz von Bilddaten; und/oder
Korrigieren, Bestätigen und/oder Zurückweisen der Ausgabedaten, die durch den die Bildanalyseaufgabe an der Instanz von Bilddaten durchführenden Maschinenlernalgorithmus erzeugt werden; und
Modifizieren eines oder mehrerer Parameter des Maschinenlernalgorithmus unter Verwendung des mindestens einen Zeitdauermaßes (130), wobei der Schritt des Modifizierens das Trainieren oder erneute Trainieren des Maschinenlernalgorithmus unter Verwendung des einen oder der mehreren Zeitdauermaße umfasst, wobei die Bilddaten ein Bild sind, die Bildanalyseaufgabe eine Segmentierungsaufgabe ist und die Ausgabedaten eine Segmentierung des Bildes umfassen und der Schritt des Trainierens oder erneuten Trainierens des Maschinenlernalgorithmus Folgendes umfasst:
Erhalten der Ausgabedaten, die durch Verarbeiten der Instanz von Bilddaten unter Verwendung des Maschinenlernalgorithmus erzeugt werden (210);
Erhalten von Ground-Truth-Ausgabedaten, die eine Ground-Truth-Version der Ausgabedaten, die repräsentativ für das vorhergesagte Ergebnis der Bildanalyseaufgabe sind, repräsentieren (220);
Bestimmen eines Verlustmaßes zwischen den Ausgabedaten und den Ground-Truth-Ausgabedaten (230);
Gewichten des Verlustmaßes unter Verwendung des mindestens einen Zeitdauermaßes für die Instanz von Bilddaten (240); und
Trainieren oder erneutes Trainieren des Maschinenlernalgorithmus unter Verwendung aller gewichteten Verlustmaße (250).

2. Verfahren nach Anspruch 1, das ferner Normieren des mindestens einen erhaltenen Zeitdauermaßes umfasst, bevor für eine Instanz von Bilddaten das Verlustmaß gewichtet wird (340).

3. Verfahren nach Anspruch 2, wobei für eine Instanz von Bilddaten:
mindestens ein Zeitdauermaß für die Instanz von Bilddaten ein Maß für die Zeit ist, die die Person benötigt, um die Bildanalyseaufgabe für die Instanz von Bilddaten durchzuführen; und
die Ground-Truth-Ausgabedaten Daten sind, die von der die Bildanalyseaufgabe für die Instanz von Bilddaten durchführenden Person erzeugt werden.

4. Verfahren nach Anspruch 2 oder 3, wobei der Schritt des Bestimmens eines Verlustmaßes Bestimmen eines Kreuzentropie-Verlustmaßes und/oder eines Dice-Verlustmaßes zwischen den Ausgabedaten und den Ground-Truth-Ausgabedaten umfasst (230).

5. Verfahren nach einem der Ansprüche 1 bis 4, wobei das mindestens eine Zeitdauermaß für jede von einer oder mehreren Instanzen von Bilddaten ein Maß für die Zeit umfasst, die die Person aufwendet, um Ausgabedaten zu ergänzen, die durch den Maschinenlernalgorithmus erzeugt werden, nachdem die Instanz von Bilddaten verarbeitet worden ist, um die Bildanalyseaufgabe an der Instanz von Bilddaten durchzuführen.

6. Verfahren nach einem der Ansprüche 1 bis 5, wobei das mindestens eine Zeitdauermaß für eine Instanz von Bilddaten ein Maß für die Zeit umfasst, die die Person aufwendet, um Ausgabedaten zu bestätigen, die durch den Maschinenlernalgorithmus erzeugt werden, nachdem die Instanz von Bilddaten verarbeitet worden ist, um die Bildanalyseaufgabe an der Instanz von Bilddaten durchzuführen.

7. Verfahren nach einem der Ansprüche 1 bis 6, wobei das mindestens eine Zeitdauermaß für eine Instanz von Bilddaten ein Maß für die Zeit umfasst, die die Person aufwendet, um mindestens einen Teil von Ausgabedaten zu entfernen, die durch den Maschinenlernalgorithmus erzeugt werden, nachdem die Instanz von Bilddaten verarbeitet worden ist, um die Bildanalyseaufgabe an der Instanz von Bilddaten durchzuführen.

8. Verfahren nach einem der Ansprüche 1 bis 7, wobei:
die Bildanalyseaufgabe eine Bildklassifizierungsaufgabe ist;
der Maschinenlernalgorithmus einen Schwellenwert verwendet, um vorherzusagen, ob die Bilddaten zu einer Klassifizierung gehören oder nicht; und wobei
das Modifizieren eines oder mehrerer Parameter des Maschinenlernalgorithmus Verwenden des einen oder der mehreren Zeitdauermaße umfasst, um den Wert des Schwellenwerts zu modifizieren (130).

9. Verfahren nach einem der Ansprüche 1 bis 8, wobei die Instanz von Bilddaten mindestens 50 Instanzen von Bilddaten umfasst.

10. Computerimplementiertes Verfahren nach einem der Ansprüche 1 bis 9, wobei der Maschinenlernalgorithmus ein faltendes neuronales Netz ist.

11. Computerprogramm, das Computerprogrammcodemittel umfasst, die, wenn sie in einer Rechenvorrichtung mit einem Verarbeitungssystem ausgeführt werden, bewirken, dass das Verarbeitungssystem Schritte des Verfahrens nach einem der Ansprüche 1 bis 10 durchführt.

12. Verarbeitungssystem zum Modifizieren eines Maschinenlernalgorithmus, der zum Verarbeiten von Bilddaten zum Durchführen einer Bildanalyseaufgabe und zum Erzeugen von Ausgabedaten, die repräsentativ für ein vorhergesagtes Ergebnis der Bildanalyseaufgabe sind, ausgelegt ist,
wobei das Verarbeitungssystem für Folgendes ausgelegt ist:
Erhalten mindestens eines Zeitdauermaßes für eine oder mehrere Instanzen von Bilddaten, wobei jedes Zeitdauermaß ein Maß für die Zeit ist, die eine Person für Folgendes benötigt:
Durchführen der Bildanalyseaufgabe für die Instanz von Bilddaten; und/oder
Korrigieren, Bestätigen oder Zurückweisen der Ausgabedaten, die durch den die Bildanalyseaufgabe an der Instanz von Bilddaten durchführenden Maschinenlernalgorithmus erzeugt werden; und
Modifizieren eines oder mehrerer Parameter des Maschinenlernalgorithmus unter Verwendung des einen oder der mehreren Zeitdauermaße, wobei das Modifizieren Trainieren oder erneutes Trainieren des Maschinenlernalgorithmus unter Verwendung des einen oder der mehreren Zeitdauermaße umfasst,
wobei die Bilddaten ein Bild sind, die Bildanalyseaufgabe eine Segmentierungsaufgabe ist und die Ausgabedaten eine Segmentierung des Bildes umfassen und wobei das Trainieren oder erneute Trainieren des Maschinenlernalgorithmus für Folgendes ausgelegt ist:
Erhalten der Ausgabedaten, die durch Verarbeiten der Instanz von Bilddaten unter Verwendung des Maschinenlernalgorithmus erzeugt werden;
Erhalten von Ground-Truth-Ausgabedaten, die eine Ground-Truth-Version der Ausgabedaten, die repräsentativ für das vorhergesagte Ergebnis der Bildanalyseaufgabe sind, repräsentieren;
Bestimmen eines Verlustmaßes zwischen den Ausgabedaten und den Ground-Truth-Ausgabedaten; und
Gewichten des Verlustmaßes unter Verwendung des mindestens einen Zeitdauermaßes für die Instanz von Bilddaten (240); und
Trainieren oder erneutes Trainieren des Maschinenlernalgorithmus unter Verwendung aller gewichteten Verlustmaße.

## Revendications

1. Procédé mis en œuvre par ordinateur (100) pour traiter des données d'image afin d'effectuer une tâche d'analyse d'image et produire des données de sortie représentatives d'un résultat prédit de la tâche d'analyse d'image, le procédé comprenant :
l'obtention d'au moins une mesure de temps (120) pour une ou plusieurs instances de données d'image, la mesure de temps étant une mesure du temps nécessaire à un individu pour :
effectuer la tâche d'analyse d'image pour l'instance de données d'image ; et/ou
corriger, approuver et/ou désapprouver les données de sortie produites par l'algorithme d'apprentissage automatique effectuant la tâche d'analyse d'image sur l'instance de données d'image ; et
la modification d'un ou plusieurs paramètres de l'algorithme d'apprentissage automatique à l'aide de l'au moins une mesure de temps (130), l'étape de modification comprenant l'entraînement ou le réentraînement de l'algorithme d'apprentissage automatique à l'aide de la ou des mesures de temps, les données d'image étant une image, la tâche d'analyse d'image étant une tâche de segmentation et les données de sortie comprenant une segmentation de l'image et l'étape d'entraînement ou de réentraînement de l'algorithme d'apprentissage automatique comprenant :
l'obtention des données de sortie produites par traitement de l'instance de données d'image à l'aide de l'algorithme d'apprentissage automatique (210) ;
l'obtention de données de sortie de vérité terrain représentant une version de vérité terrain des données de sortie représentatives du résultat prédit de la tâche d'analyse d'image (220) ;
la détermination d'une mesure de perte entre les données de sortie et les données de sortie de vérité terrain (230) ;
la pondération de la mesure de perte à l'aide de ladite au moins une mesure de temps pour l'instance de données d'image (240) ; et
l'entraînement ou le réentraînement de l'algorithme d'apprentissage automatique à l'aide de toutes les mesures de perte pondérées (250).

2. Procédé selon la revendication 1, comprenant en outre la normalisation de l'au moins une mesure de temps obtenue avant la pondération, pour l'instance de données d'image, de la mesure de perte (340).

3. Procédé selon la revendication 2, dans lequel, pour l'instance de données d'image :
au moins une mesure du temps pour l'instance de données d'image est une mesure du temps nécessaire à l'individu pour effectuer la tâche d'analyse d'image pour l'instance de données d'image ; et
les données de sortie de vérité terrain sont des données produites par l'individu effectuant la tâche d'analyse d'image pour l'instance de données d'image.

4. Procédé selon la revendication 2 ou 3, dans lequel l'étape de détermination d'une mesure de perte comprend la détermination d'une mesure de perte d'entropie croisée et/ou d'une mesure de perte de Dice entre les données de sortie et les données de sortie de vérité terrain (230).

5. Procédé selon l'une quelconque des revendications 1 à 4, dans lequel l'au moins une mesure de temps, pour chacune d'une ou plusieurs instances de données d'image, comprend une mesure du temps mis par l'individu pour compléter les données de sortie produites par l'algorithme d'apprentissage automatique après le traitement de l'instance de données d'image pour effectuer la tâche d'analyse d'image sur l'instance de données d'image.

6. Procédé selon l'une quelconque des revendications 1 à 5, dans lequel l'au moins une mesure de temps, pour l'instance de données d'image, comprend une mesure du temps mis par l'individu pour approuver des données de sortie produites par l'algorithme d'apprentissage automatique après le traitement de l'instance de données d'image pour effectuer la tâche d'analyse d'image sur l'instance de données d'image.

7. Procédé selon l'une quelconque des revendications 1 à 6, dans lequel l'au moins une mesure de temps, pour l'instance de données d'image, comprend une mesure du temps mis par l'individu pour éliminer au moins une partie des données de sortie produites par l'algorithme d'apprentissage automatique après le traitement de l'instance de données d'image pour effectuer la tâche d'analyse d'image sur l'instance de données d'image.

8. Procédé selon l'une quelconque des revendications 1 à 7, dans lequel :
la tâche d'analyse d'image est une tâche de classification d'image ;
l'algorithme d'apprentissage automatique utilise un seuil pour prédire si les données d'image appartiennent ou non à une classification ; et dans lequel
la modification d'un ou plusieurs paramètres de l'algorithme d'apprentissage automatique comprend l'utilisation de la ou des mesures de temps pour modifier la valeur du seuil (130).

9. Procédé selon l'une quelconque des revendications 1 à 8, dans lequel l'instance de données d'image comprend au moins 50 instances de données d'image.

10. Procédé mis en œuvre par ordinateur selon l'une quelconque des revendications 1 à 9, dans lequel l'algorithme d'apprentissage automatique est un réseau de neurones convolutif.

11. Programme informatique comprenant des moyens de code de programme informatique qui, lorsqu'ils sont exécutés sur un dispositif informatique comportant un système de traitement, amènent le système de traitement à effectuer des étapes du procédé selon l'une quelconque des revendications 1 à 10.

12. Système de traitement pour modifier un algorithme d'apprentissage automatique configuré pour traiter des données image afin d'effectuer une tâche d'analyse d'image et produire des données de sortie représentatives d'un résultat prédit de la tâche d'analyse d'image,
le système de traitement étant configuré pour :
obtenir au moins une mesure de temps pour une ou plusieurs instances de données d'image, chaque mesure de temps étant une mesure du temps nécessaire à un individu pour :
effectuer la tâche d'analyse d'image pour l'instance de données d'image ; et/ou
corriger, approuver ou désapprouver les données de sortie produites par l'algorithme d'apprentissage automatique effectuant la tâche d'analyse d'image sur l'instance des données d'image ; et
modifier un ou plusieurs paramètres de l'algorithme d'apprentissage automatique à l'aide de la ou des mesures de temps, la modification comprenant l'entraînement ou le réentraînement de l'algorithme d'apprentissage automatique à l'aide de la ou des mesures de temps,
dans lequel les données d'image sont une image, la tâche d'analyse d'image est une tâche de segmentation, et les données de sortie comprennent une segmentation de l'image, et dans lequel l'entraînement ou le réentraînement de l'algorithme d'apprentissage automatique est configuré pour :
obtenir les données de sortie produites par traitement de l'instance de données d'image à l'aide de l'algorithme d'apprentissage automatique ;
obtenir des données de sortie de vérité terrain représentant une version de vérité terrain des données de sortie représentatives du résultat prédit de la tâche d'analyse d'image ;
déterminer une mesure de perte entre les données de sortie et les données de sortie de vérité terrain ; et
pondérer la mesure de perte à l'aide de l'au moins une mesure de temps pour l'instance de données d'image (240) ; et
entraîner ou réentraîner l'algorithme d'apprentissage automatique à l'aide de toutes les mesures de perte pondérées.
